(19) Europäisches Patentamt
European Patent Office
Office européen des brevets

(11) **EP 4 354 899 A1**

(12) **EUROPEAN PATENT APPLICATION**

(43) Date of publication:
**17.04.2024 Bulletin 2024/16**

(21) Application number: **23197871.9**

(22) Date of filing: **18.09.2023**

(51) International Patent Classification (IPC):
*H04R 3/04* (2006.01)    *H04R 29/00* (2006.01)
*A61B 5/12* (2006.01)    *H04R 25/00* (2006.01)

(52) Cooperative Patent Classification (CPC):
**H04R 29/001; A61B 5/125; H04R 3/04;** H04R 25/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **13.10.2022 GB 202215085**

(71) Applicant: **Nokia Technologies Oy
02610 Espoo (FI)**

(72) Inventors:
• **DEMIREL, Berken Utku
40100 Kirsehir (TR)**
• **AL-NAIMI, Khaldoon
Beaconsfield, HP9 1RD (GB)**
• **MONTANARI, Alessandro
Cambridge, CB1 8DZ (GB)**

(74) Representative: **Nokia EPO representatives
Nokia Technologies Oy
Karakaari 7
02610 Espoo (FI)**

(54) **APPARATUS, METHODS AND COMPUTER PROGRAMS FOR PROVIDING SIGNALS FOR OTOACOUSTIC EMISSION MEASUREMENTS**

(57)    Examples of the disclosure relate to providing signals for otoacoustic emission measurements for playback by a single loudspeaker. In examples of the disclosure an apparatus is configured to provide an audio signal. The audio signal comprises two or more frequencies and is configured to be played back by a loudspeaker to enable otoacoustic emissions within a user's ear to be measured. In examples of the disclosure at least one interference component in an output of the loudspeaker playing back the obtained audio signal is estimated. The estimated interference component can then be used to estimate a modification to be applied to at least one frequency of the audio signal to reduce the at least one interference component in the output of the loudspeaker.

*FIG. 3*

**Description**

TECHNOLOGICAL FIELD

Examples of the disclosure relate to apparatus, methods and computer programs for providing signals for otoacoustic emission measurements. Some relate to apparatus, methods and computer programs for providing signals for otoacoustic emission measurements for playback by a single loudspeaker.

BACKGROUND

Otoacoustic emissions (OAEs) are weak acoustic signals that are emitted from outer hair cells of the cochlea. The OAEs can be emitted spontaneously or in response to sound stimulation. OAEs can be measured to evaluate the hearing level of a subject.

BRIEF SUMMARY

According to various, but not necessarily all, examples of the disclosure there may be provided an apparatus comprising means for:

provided an audio signal wherein the audio signal comprises two or more frequencies and is configured to be played back by a loudspeaker to enable otoacoustic emissions within a user's ear to be measured;
estimating at least one interference component in an output of the loudspeaker playing back the obtained audio signal; and
using the estimated at least one interference component to estimate a modification to be applied to at least one frequency of the audio signal to reduce the at least one interference component in the output of the loudspeaker.

The at least one interference component may be estimated from an output of a model of the loudspeaker.
The at least one interference component may be estimated by processing a subset of a frequency range of the output of the model of the loudspeaker.
A machine learning program may be used to model the loudspeaker.
The machine learning program may comprise a neural network.
The at least one interference component may comprise at least one of: a magnitude response; or a phase response.
The at least one interference component may comprise an intermodulation distortion
Estimating the modification to be applied to at least one frequency of the audio signal may comprise determining a coefficient for multiplying at least one frequency of the audio signal.
A machine learning program may be used to estimate the modification to be applied to at least one frequency of the audio signal.
The machine learning program may comprises a neural network.
The means may be for enabling the modification to be applied to an audio signal.
The at least one loudspeaker may be configured to playback an audio signal wherein the audio signal comprises two or more frequencies and is configured to be played back by the at least one loudspeaker to enable otoacoustic emissions within a user's ear to be measured and wherein the audio signal has been modified to reduce at least one interference component.
According to various, but not necessarily all, examples of the disclosure there may be provided a method comprising:

providing an audio signal wherein the audio signal comprises two or more frequencies and is configured to be played back by a loudspeaker to enable otoacoustic emissions within a user's ear to be measured;
estimating at least one interference component in an output of the loudspeaker playing back the obtained audio signal; and
using the estimated at least one interference component to estimate a modification to be applied to at least one frequency of the audio signal to reduce the at least one interference component in the output of the loudspeaker.

According to various, but not necessarily all, examples of the disclosure there may be provided a computer program comprising instructions which, when executed by an apparatus, cause the apparatus to perform at least:

providing an audio signal wherein the audio signal comprises two or more frequencies and is configured to be played back by a loudspeaker to enable otoacoustic emissions within a user's ear to be measured;

estimating at least one interference component in an output of the loudspeaker playing back the obtained audio signal; and

using the estimated at least one interference component to estimate a modification to be applied to at least one frequency of the audio signal to reduce the at least one interference component in the output of the loudspeaker.

While the above examples of the disclosure and optional features are described separately, it is to be understood that their provision in all possible combinations and permutations is contained within the disclosure. It is to be understood that various examples of the disclosure can comprise any or all of the features described in respect of other examples of the disclosure, and vice versa. Also, it is to be appreciated that any one or more or all of the features, in any combination, may be implemented by/comprised in/performable by an apparatus, a method, and/or computer program instructions as desired, and as appropriate.

BRIEF DESCRIPTION

Some examples will now be described with reference to the accompanying drawings in which:

FIGS. 1A and 1B show amplitude spectrums;
FIG. 2 shows an example method;
FIG. 3 shows an example system;
FIG. 4 shows training of an example loudspeaker model;
FIG. 5 shows training of an example correction model;
FIG. 6 shows results obtained for examples of the disclosure;
FIGS. 7A and 7B show results obtained for examples of the disclosure;
FIG. 8 shows example devices according to examples of the disclosure; and
FIG. 9 shows an apparatus according to examples of the disclosure.

The figures are not necessarily to scale. Certain features and views of the figures can be shown schematically or exaggerated in scale in the interest of clarity and conciseness. For example, the dimensions of some elements in the figures can be exaggerated relative to other elements to aid explication. Corresponding reference numerals are used in the figures to designate corresponding features. For clarity, all reference numerals are not necessarily displayed in all figures.

DETAILED DESCRIPTION

Otoacoustic emissions (OAEs) are useful for evaluating the hearing level of a subject because their measurement does not require any active cooperation from the subject. Unlike other methods, such as audiometry there is no need to obtain any feedback from a user. For example, there is no need for the subject to actuate a button or provide any feedback indicating how well they can hear.

To measure OAEs otoacoustic signals are played into the ear of the subject and the response can be detected by one or more microphones.

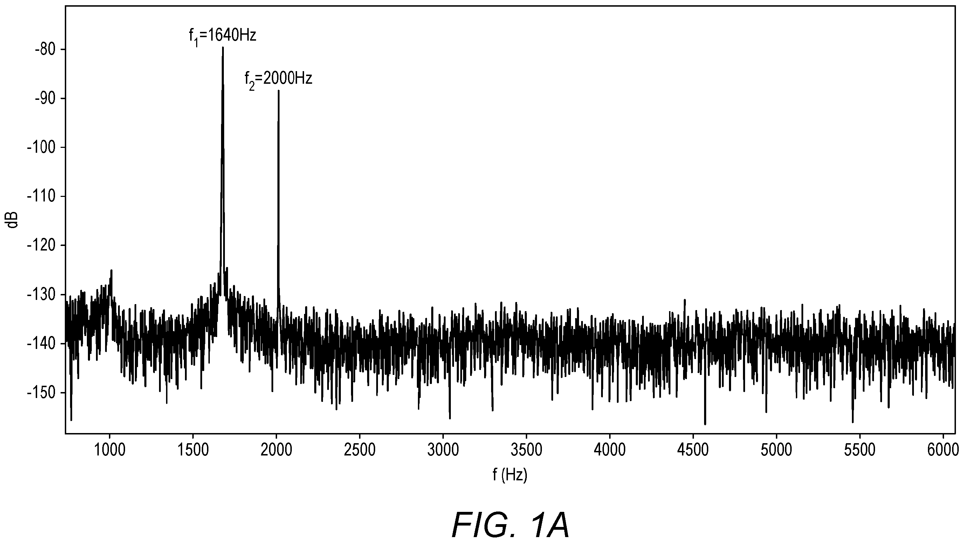

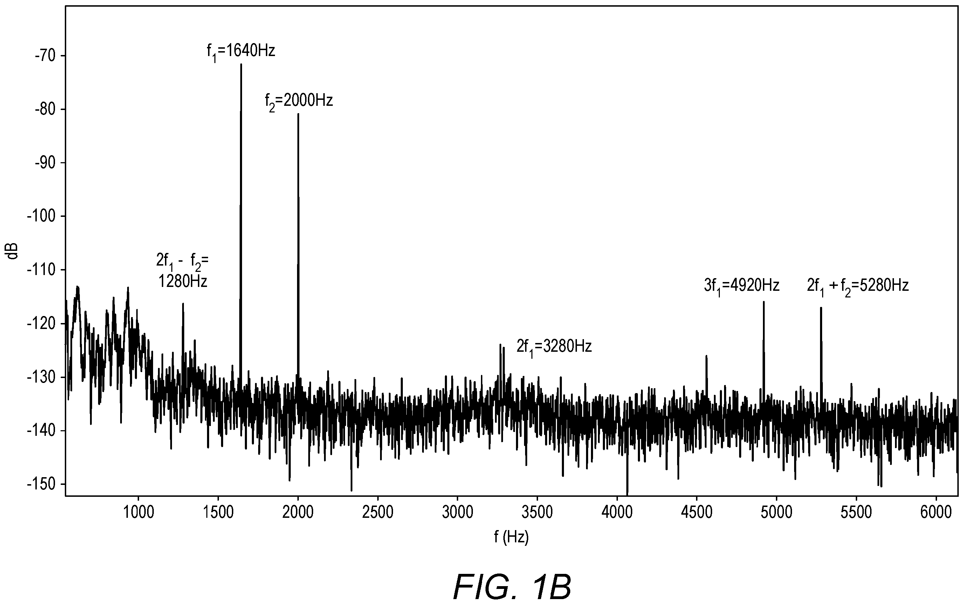

Figs. 1A and 1B show amplitude spectrums for the outputs of loudspeakers playing a pair of tones that can be used for measuring otoacoustic emissions.

Fig. 1A shows an amplitude spectrum for two loudspeakers playing the pair of tones.

The x axis plots the frequency in Hz and the y axis plots the amplitude in dB. The audio signals being played back comprise two frequency components or tones. The first frequency component has a frequency $f_1$ of 1640Hz and the second frequency component has a frequency $f_2$ of 2000Hz. These two frequencies are examples of frequencies that could be used in audio signals for otoacoustic measurements. A plurality of different pairs of frequencies would be used to make the otoacoustic measurements.

The different frequency components have different amplitudes. In this example the first frequency component has a larger amplitude than the second frequency component.

The first frequency component can be played back by a first loudspeaker and the second frequency component can be played back by a second loudspeaker.

As shown in Fig. 1A there are no significant distortions when the pair of tones for otoacoustic measurements are played by two loudspeakers. There are no distortions with an amplitude above the general noise level.

Fig. 1B shows an amplitude spectrum for a single loudspeaker playing the pair of tones. The x axis plots the frequency in Hz and the y axis plots the amplitude in dB.

The audio signals being played back comprise two frequency components or tones. The first frequency com-

ponent has a frequency $f_1$ of 1640Hz and the second frequency component has a frequency $f_2$ of 2000Hz. These two frequencies are examples of frequencies that could be used in audio signals for otoacoustic measurements. A plurality of different pairs of frequencies would be used to make the otoacoustic measurements.

The different frequency components have different amplitudes. In this example the first frequency component has a larger amplitude than the second frequency component.

In this example both the first frequency component and the second frequency component are played back by the same loudspeaker.

As shown in Fig. 1B there are significant intermodulation distortions. The intermodulation distortions arise due to the non-linearity of the loudspeaker. The intermodulation distortions arise due to the interaction of the respective frequency components with each other.

The example of Fig. 1B shows that there are multiple intermodulation distortions with an amplitude above the general noise level. In the example shown in Fig. 1B there are intermodulation distortions at $2f_1$-$f_2$, $2f_1$, $3f_1$, $2f1+f2$ that have an amplitude larger than the noise level.

The intermodulation distortion at $2f_1$-$f_2$ occurs at the same frequency as the cochlea response that is used for distortion product OAE measurements. This intermodulation distortion caused by the non-linearity of the loudspeaker therefore prevents the OAE measurements being made because a microphone would detect the distortion over the emission from the inner ear.

The effect of intermodulation distortions can be particularly pronounced for devices such as earbuds which have to be small enough to fit into a user's ear and so only have space for a small loudspeaker. The small loudspeakers have limitations on the movement of the cone and so can show higher non-linear behaviours than bigger loudspeakers.

Examples of this disclosure provide methods and apparatus for removing or reducing the intermodulation distortions and so enabling devices with a single loudspeaker to be used for OAE measurements. The example methods and apparatus are energy and memory efficient for measuring the OAEs and have a low latency. This can enable the methods to be used in real time while the OAEs are being measured.

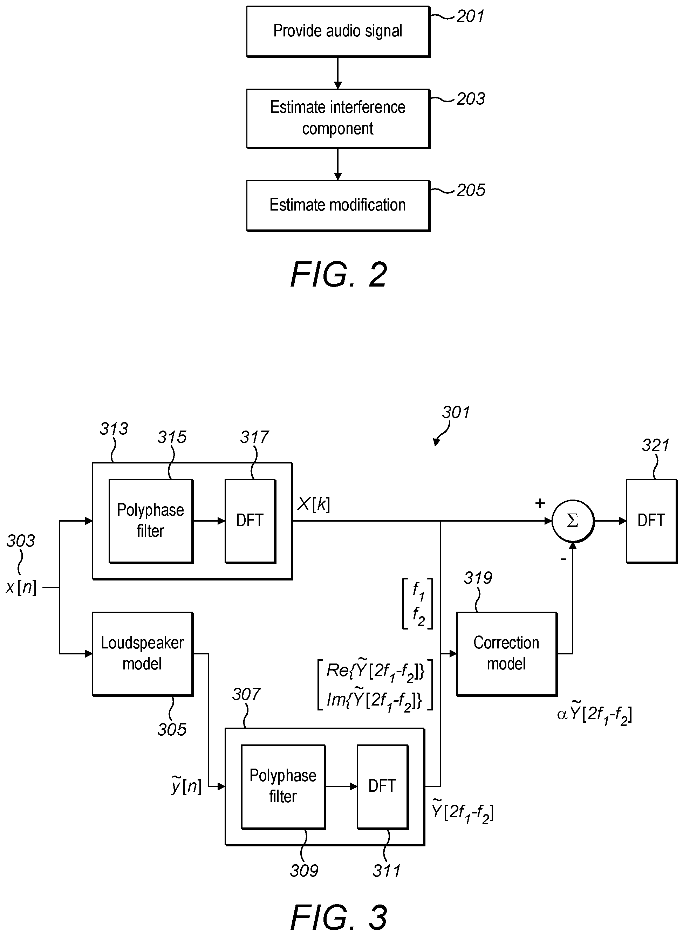

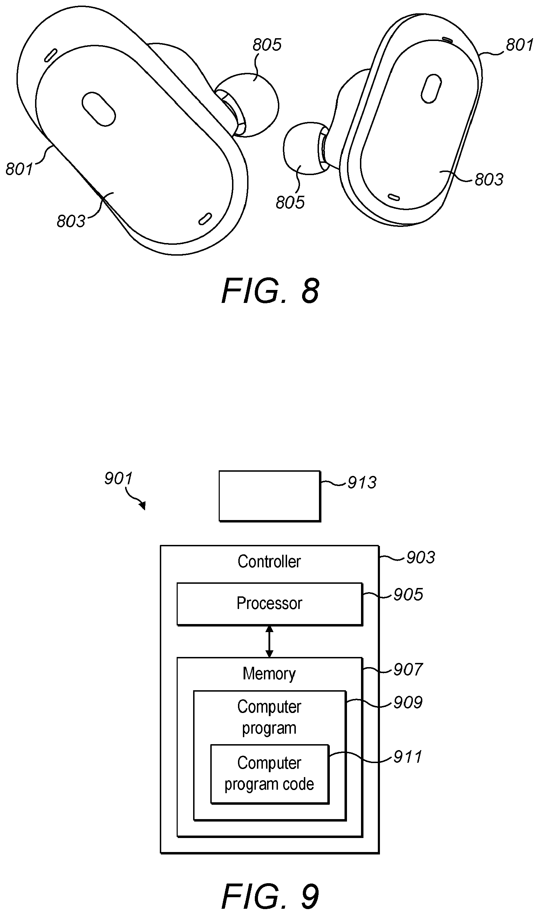

Fig. 2 shows an example method according to examples of the disclosure. The method could be implemented using any suitable apparatus or device. An example apparatus that could be used to implement examples of the disclosure is shown below in Fig. 9.

The method comprises, at block 201, providing an audio signal. The audio signal can be provided by being retrieved from a memory or could be received from another device or could be provided in any other suitable manner. The audio signal is an electrical input signal that can be provided as an input to a loudspeaker and played back the loudspeaker.

The audio signal comprises two frequencies. The audio signal comprises a first component at a first frequency and a second component at a second frequency. The different frequency components have different amplitudes. The different frequency components provide a two-tone signal.

The audio signal is configured to be played back by a loudspeaker to enable otoacoustic emissions within a user's ear to be measured. In examples of the disclosure a single loudspeaker could be used to playback the audio signal. The loudspeaker that is used to playback the audio signal could be a non-linear loudspeaker. The loudspeaker could be a small loudspeaker such as a loudspeaker within an earbud or other suitable inner ear device.

In some examples the audio signals can comprise a sinusoidal waveform. Other types of waveform could be used in other examples.

At block 203 the method comprises estimating at least one interference component in an output of the loudspeaker playing back the audio signal that was obtained at block 203. The interference component could be an intermodulation distortion or any other suitable type of interference. The interference conference component can be caused by non-linearities of the loudspeaker or any other suitable cause.

Any suitable means could be used to estimate the interference component. In some examples the interference component can be estimated from an output of a model of the loudspeaker.

Any suitable means could be used to model the loudspeaker. In some examples a machine learning program or other rules based algorithm can be used to model the loudspeaker. The machine learning program could be neural network or any other suitable type of machine learning program. The model of the loudspeaker can be configured to receive an audio signal as an input and provide a predicted loudspeaker response as an output. Different models could be used for different types of loudspeakers and/or different devices that comprise the loudspeaker.

The predicted response of the loudspeaker can be used to estimate the interference component. In some examples the interference component can be estimated by analysing or processing a part of the output of the loudspeaker. In some examples the interference component can be estimated by processing a subset of a frequency range of the output of the model of the loudspeaker. The part of the output of the loudspeaker that is to be analysed or processed can be obtained by applying a polyphase filter, or any other suitable type of filter, to the output of the model of the loudspeaker. The polyphase filter, or other suitable type of filter, can be configured to select the frequencies at which the interference component occurs. For example, the filter could be configured to select the $2f_1$-$f_2$ components of the

signal or other components that occur at the same or similar frequency as the cochlea response.

In some examples a waveform of the interference component is estimated. In some examples the estimated interference component comprises a magnitude response and/or a phase response and/or any other suitable parameter.

At block 205 the method comprises using the estimated interference component to estimate a modification to be applied to at least one frequency of the audio signal to reduce the interference component in the output of the loudspeaker.

Any suitable means can be used to estimate the modification that is to be applied to the audio signal. In some examples a machine learning program or other rules based algorithm can be used to estimate the modification that is to be applied to the audio signal. The machine learning program could comprise a neural network or any other suitable type of program.

In some examples the modification can be estimated using the interference component that was estimated at block 203. For instance, the estimated interference component, or data derived from the estimated interference component, could be used as an input to a machine learning program and used to estimate a modification.

In some examples the modification that is estimated can comprise a coefficient that is to be applied to at least one component or frequency of the audio signal. The coefficient can be a correction coefficient. The correction coefficient could be applied to the first component or frequency of the audio signal and/or a second component or frequency of the audio signal. For instance, a first component of the audio signal could be multiplied by the correction coefficient and could then be subtracted from the audio signal.

In examples of the disclosure the estimated modification can be applied to an audio signal to provide a modified audio signal. The modified audio signal comprises a first frequency component, a second frequency component and a component comprising the modification. The modified audio signal can be played back by the loudspeaker. The modified audio signal has a reduced interference component. For example, the modified audio signal could have a reduced intermodulation distortion at $2f_1$-$f_2$, the frequency of the cochlea response that is used for distortion product OAE measurements.

Fig. 3 shows an example system 301. The system 301 could be used to implement methods such as the method of Fig. 2 or any other suitable methods or variations of the method. The method could be implemented using an apparatus 901 as shown in Fig. 9 or any other suitable type of apparatus or combinations of apparatus.

The system 301 is configured to obtain an audio signal 303 as an input. The audio signal 303 is denoted $x[n]$ in Fig. 3. The audio signal 303 is configured to be played back by a loudspeaker to enable otoacoustic emissions within a user's ear to be measured. The audio signal 303 can be an electrical signal that could be provided as an input to a loudspeaker to enable the audio signal to be played back by the loudspeaker and used for measuring otoacoustic emissions.

The audio signal 303 can comprise a sinusoidal waveform comprising two frequencies. The audio signal 303 comprises a first component at a first frequency $f_1$ and a second component at a second frequency $f_2$. The different frequency components have different amplitudes. Other types of waveform could be used in other examples.

In the example of Fig. 3 the audio signal 303 is provided as an input to a loudspeaker model 305. The loudspeaker model 305 is a model of a single loudspeaker. The loudspeaker model 305 can be a model of a non-linear loudspeaker. The non-linear loudspeaker could be a loudspeaker as used in an inner ear device such as an earbud, or any other suitable type of loudspeaker. Different loudspeaker models 305 could be used to predict outputs for different types of loudspeaker and/or for different configurations of the loudspeakers.

The loudspeaker model 305 can be a computational model. The loudspeaker model 305 could comprise a machine learning program such as a neural network or any other suitable type of program.

The loudspeaker model 305 is configured to receive the audio signal 303 as an input and provide an estimate of the outputs of the loudspeaker as an output. The estimated output of the loudspeaker is denoted $\tilde{y}[n]$ in Fig. 3.

The system 301 is configured so that the estimated output of the loudspeaker $\tilde{y}[n]$ is provided as an input to a distortion prediction module 307. The distortion prediction module 307 is configured to process the estimated output of the loudspeaker $\tilde{y}[n]$ to provide a predicted intermodulation distortion as an output. The predicted intermodulation distortion is denoted as $\tilde{y}[2f_1 - f_2]$. Other types of interference could be estimated in other examples.

In the example of Fig. 3 the distortion prediction module 307 comprises a polyphase filter 309 and discrete Fourier transform module 311. Other means for estimating the intermodulation distortion or other type of interference could be used in other examples.

The polyphase filter 309 is configured to select a part of the output of the loudspeaker model 305 for analysis or processing. The polyphase filter 309 is configured to filter selected phases of the estimated output of the loudspeaker $\tilde{y}[n]$. The polyphase filter 305 can be configured so that the analysis or processing is focused on the frequency ranges at which intermodulation distortion occurs. In the example of Fig. 3 the polyphase filter 309 can be configured to limit the signal to the frequency components at which the intermodulation distortion occurs. In some examples the polyphase filter 309 can be configured to limit the signal to the $2f_1$-$f_2$ as this is where the problematic intermodulation distortion occurs.

The discrete Fourier transform module 311 is configured to transform the filtered signal from the time domain to a frequency domain. The transformation to the frequency domain enable phase and magnitude information relating to the intermodulation distortion to be obtained. Other types of transform could be used in other examples of the disclosure. The discrete Fourier transform module 311 can enable real and imaginary parts of the intermodulation distortion to be predicted.

The distortion prediction module 307 provides the predicted intermodulation distortion as an output. The predicted intermodulation distortion can be denoted $\tilde{Y}[2f_1 - f_2]$. The predicted intermodulation distortion has a real part and an imaginary part which can be denoted respectively as: $Re\{\tilde{Y}[2f_1 - f_2]\}$ and $Im\{\tilde{Y}[2f_1 - f_2]\}$.

In the system 301 of Fig. 3 the predicted intermodulation distortion $\tilde{Y}[2f_1 - f_2]$ is provided as an input to a correction model 319.

The system of Fig. 3 is also configured so that the audio signal 303 is provided as an input to a transformation module 313. The transformation module 313 can be configured to transform the audio signal 303, or part of the audio signal 303, from the time domain to a frequency domain.

In the example of Fig. 3 the transformation module 313 comprises a polyphase filter 315 and discrete Fourier transform module 317. The polyphase filter 315 and discrete Fourier transform module 317 can perform the same processes as the polyphase filter 309 and discrete Fourier transform module 311 of the distortion prediction module 307. This can enable the same transformation to be applied to both the audio signal 303 and the estimated output of the loudspeaker $\tilde{y}[n]$.

The transformation module 313 provides a frequency domain audio signal $X[k]$ as an output. The frequency domain audio signal comprises a first component at the first frequency $f_1$ and a second component at the second frequency $f_2$.

The correction model 319 receives the frequency domain audio signal and the predicted intermodulation distortion as an input. The correction model 319 is configured to use the predicted intermodulation distortion and the frequency domain audio signal to estimate a correction or modification that can be applied to the audio signal so as to reduce the intermodulation distortion in the loudspeaker output.

In the example of Fig. 3 the correction model 319 receives a vector input wherein the values of the vector correspond to the respective components $f_1$, $f_2$ and the real and imaginary parts of the predicted intermodulation distortion $Re\{\tilde{Y}[2f_1 - f_2]\}$ and $Im\{\tilde{Y}[2f_1 - f_2]\}$. Other inputs and/or formats of the inputs could be used in other examples.

The correction model 319 is configured to process the input to estimate a correction or modification that can be applied to the audio signal 303. The correction model 319 can be a computational model. The correction model 319 could comprise a machine learning program such as a neural network or any other suitable type of program.

The output provided by the correction model 319 can comprise a correction coefficient or other suitable parameter as an output. The correction coefficient can comprise a value that is applied to the intermodulation distortion and then subtracted from the audio signal 303. In the example of Fig. 3 the correction coefficient comprises the value $\alpha$. The predicted intermodulation distortion can be multiplied by the value of $\alpha$ to generate $\alpha\tilde{Y}[2f_1 - f_2]$ which is a modification that can be applied to the audio signal 303. The modification is an additional sinusoidal frequency at the same frequency as the intermodulation distortion. The phase of the modification is inverted relative to the audio signal 303.

In the example of Fig. 3 the modification $\alpha\tilde{Y}[2f_1 - f_2]$ is added to the frequency domain audio signal. This generates a modified audio signal comprising the first frequency component, the second frequency components, and the additional modification based on the predicted intermodulation distortion.

The system 301 is configured so that the modified audio signal is provided to an inverse transformation module 321. The inverse transformation module 321 is configured to transform the modified audio signal back to the time domain. In the example of Fig. 3 the inverse transformation module 321 comprises an inverse discrete Fourier transform. The transform that is used to covert the modified audio signal back to the time domain can correspond to the transforms that were used to transform the original audio signal and estimated output of the loudspeaker into the frequency domain.

The modified audio signal can be played back by a single loudspeaker and used for measuring otoacoustic emissions in a user's ear. As the audio signals have been modified to remove or reduce the intermodulation distortion the modified audio signals can be played back by a single, non-linear loudspeaker. This could enable inner ear devices such as ear buds, or other devices with a single small loudspeaker, to be used to measure otoacoustic emissions.

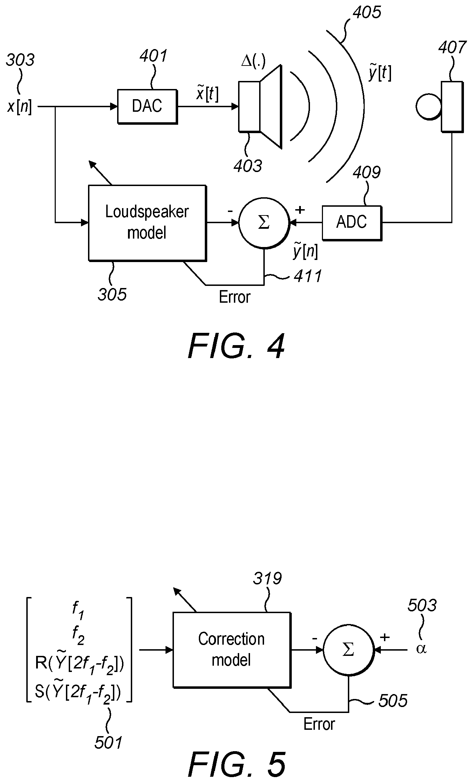

The loudspeaker model 305 and the correction model 319 can be pre-trained models. The training of these models can be performed offline. For example, the training of the models can be performed when a new measuring device is being built and designed. The trained models can then be provided for use by the respective measuring devices. Figs. 4 and 5 show example methods for training the respective models.

Fig. 4 shows training of an example loudspeaker model 305. The example loudspeaker model 305 could be as shown in Fig. 3 or could be any other suitable type of model.

The loudspeaker model 305 could comprise a machine learning program. The machine learning program can comprise a neural network or any other suitable type of trainable model. The term "Machine Learning program" refers to any kind of artificial intelligence (AI), intelligent or other method that is trainable or tunable using data. The machine learning program can comprise a computer program. The machine learning program can be trained to perform a task, such as estimating an output for a loudspeaker or determining a modification for an audio signal, without being explicitly programmed to perform that task. The machine learning program can be configured to learn from experience E with respect to some class of tasks T and performance measure P if its performance at tasks in T, as measured by P, improves with experience E. In these examples the machine learning program can often learn from reference data to make estimations on future data. The machine learning program can be also a trainable computer program. Other types of machine learning programs could be used in other examples.

The training of the machine learning program can be performed by any suitable apparatus or system. For example, the machine learning program could be trained by a system or other apparatus that has a high processing. In some examples the machine learning program could be trained by a system comprising one or more graphical processing units (GPUs) or any other suitable type of processor.

In the example training process shown in Fig. 4 the audio signal 303 is provided as an input to the loudspeaker model 305. The loudspeaker model 305 provides an estimated loudspeaker output as an output.

Before the loudspeaker model 305 is trained it can be configured in an initial state. In the initial state the weights or other parameters of the loudspeaker model 305 can be set to any suitable value. In some examples the initial weights of the loudspeaker model 305 can be random.

The audio signal 303 is also provided to a digital to analogue converter 401 to generate the analogue audio signal $\tilde{x}(t)$. The analogue audio signal $\tilde{x}(t)$ is then provided as an input to a loudspeaker 403.

The loudspeaker 403 can comprise any means that can be configured to convert an input electrical signal into an output acoustic signal 405. In this example the acoustic signal 405 comprises a pressure wave that can be detected by the outer hair cells of the user's ear. In this example the output signal is an otoacoustic signal that can be used for measuring the otoacoustic emissions of a user's ear. The output acoustic signal 405 is denoted $\tilde{y}(t)$ in this example.

The loudspeaker 403 can be a physical device that is intended for use in a device measuring otoacoustic emissions. The loudspeaker 403 can be a non-linear loudspeaker. The loudspeaker 403 could be a small loudspeaker such as a loudspeaker that could be used in an earbud or other similar device.

For the purposes of training the loudspeaker model 305 the output 405 of the loudspeaker 403 is the ground truth and the audio signal 303 is the stimuli.

To enable training of the loudspeaker model 305 one or more microphones 407 are configured to detect the acoustic signal 405 that is output from the loudspeaker 403. The one or more microphones 407 can comprise any means that can be configured to convert an acoustic input signal into a corresponding electrical output signal. The microphones 407 can be part of a digital signal processing device or any other suitable device.

The output signal from the microphone 407 is provided to an analogue to digital converter 409. The analogue to digital converter is configured to convert the analogue output from the microphone 407 into a digital signal. This then provides a measured output of the loudspeaker 403 that can be compared to the output of the loudspeaker model 305.

The comparison between the predicted output of the loudspeaker from the loudspeaker model 305 and the measured output of the loudspeaker 403 enables the output of the loudspeaker model 305 to be evaluated relative to the actual output of the loudspeaker 403. The comparison provides an error signal 411 as an output. The error signal can comprise an error value or other indication of the difference between the estimated loudspeaker output and the actual loudspeaker output.

The error value or other indication can be provided as an input to the loudspeaker model 305. One or more weights or other parameters of the loudspeaker model 305 can be adjusted in response to the error input. For example, if the loudspeaker model 305 comprises a neural network one or more weights of the neural network can be adjusted. The weights or other parameters can be updated to decrease the loss between the output of the loudspeaker model 305 and the output of the speaker 403.

The process shown in Fig. 4 can be repeated until the outputs of the loudspeaker model 305 have sufficiently converged with the output of the speaker 403. The outputs of the loudspeaker model 305 can be determined to have converged with the output of the speaker 403 if the error value is below a given threshold or if any other suitable criteria has been satisfied.

Once the outputs of the loudspeaker model 305 have been determined to be converged the loudspeaker model 305 is considered to be trained. The trained loudspeaker model 305 can then be used in systems such as the system 301 of Fig. 1 and used to estimate a distortion level in an otoacoustic signal. The trained loudspeaker model 305 can be stored in memories of measurement devices or could be stored in locations that are accessible by the measurement devices.

Fig. 5 shows training of an example correction model 319. The example correction model 319 could be as shown in Fig. 3 or could be any other suitable type of model.

The correction model 319 could comprise a machine learning program or could comprise any other suitable type of trainable model. The correction model 319 could comprise a similar type of program to the loudspeaker model 305 or could be any other suitable type of computer program.

The input to the correction model 319 comprises an input vector 501 with four values. The input vector 501 for the correction model 319 can correspond to the inputs that are used when the correction model 319 is in use, for example, in a system as shown in Fig. 3. The first value in the input vector 501 corresponds to the first frequency component $f_1$ of the audio signal 303, the second value corresponds to the second frequency component $f_2$ of the audio signal 303, the third value corresponds to the real part of the predicted intermodulation distortion $Re\{\tilde{Y}|2f_1 - f_2|\}$ and the fourth value corresponds to the imaginary part of the predicted intermodulation distortion $Im\{\tilde{Y}|2f_1 - f_2|\}$. The respective parts of the intermodulation distortion can be obtained using a loudspeaker model 305 and a system as shown in Fig. 3, or by using any other suitable means. Other values and formats for the input vector could be used in some examples.

In the example training process of Fig. 5 the correction model 319 is configured in an initial state. In the initial state the weights or other parameters of the correction model 319 can be set to any suitable value. In some examples the initial weights of the correction model 319 can be random.

A correction coefficient 503 is selected for respective otoacoustic input signals by searching over a range of coefficient values. The coefficient values can be searched over a selected range. The range of values that are used can be determined based on the non-linearity of the loudspeaker and/or any other suitable factors. In some examples the search can be carried out between the values of 0.7 to 1.7 with a 0.02 increment. The value that is selected for the correction coefficient 503 for a given input vector is the value that provides the best intermodulation distortion ratio between the modified audio signal and the original audio signal.

To train the correction model 319 an input vector 501 comprising the four values is provided as an input. The selected coefficient is the desired output, or ground truth, that should be predicted by the correction model 319. The respective inputs and the corresponding coefficients are used to form a dataset that can be used to train the correction model 319.

The correction model 319 is trained used the dataset comprising the respective inputs and the corresponding coefficients. During training the output of the correction model 319 is compared to the desired correction coefficient 503. The error between these values is provide back to the correction model 319 in an error signal 505.

One or more weights or other parameters of the correction model 319 can be adjusted in response to the error input. For example, if the correction model 319 comprises a neural network one or more weights of the neural network can be adjusted. The weights or other parameters can be updated to decrease the loss between the output of the correction model 319 and the output of the desired correction coefficient 503.

The process shown in Fig. 5 can be repeated until the outputs of the correction model 319 have sufficiently converged with the desired correction coefficient. The outputs of the correction model 319 can be determined to have converged with the desired correction coefficient 503 if the error value is below a given threshold or if any other suitable criteria has been satisfied.

Once the outputs of the correction model 319 have been determined to be converged the correction model 319 is considered to be trained. The trained correction model 319 can then be used in systems such as the system 301 of Fig. 3 and used to determine a modification for an audio signal 303 that can be used to reduce intermodulation distortions in the audio signal 303. The trained correction model 319 can be stored in memories of measurement devices or could be stored in locations that are accessible by the measurement devices.

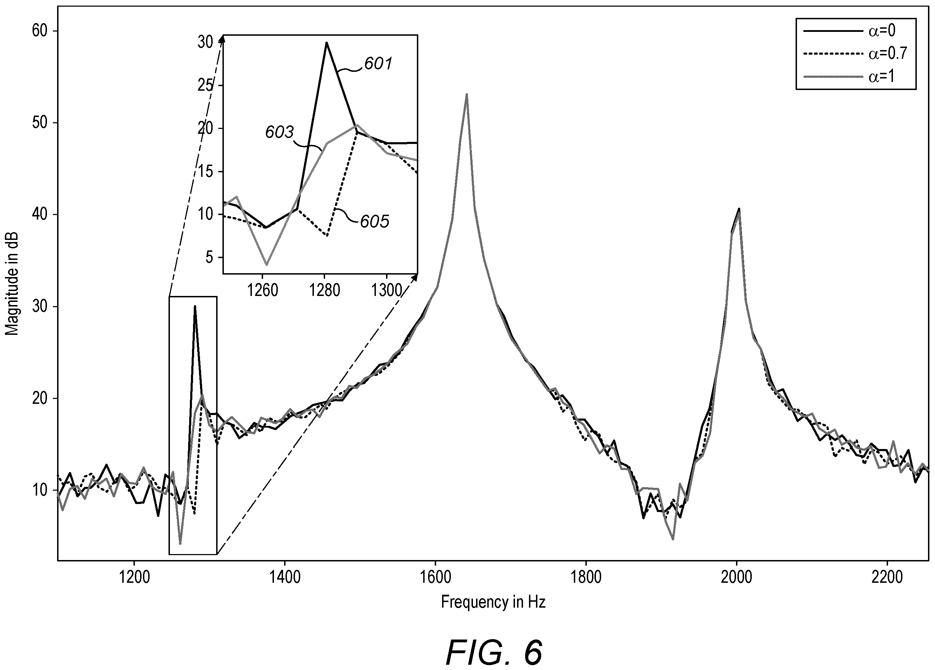

Fig. 6 shows example results obtained using examples of the disclosure. Fig. 6 shows a magnitude spectrum of a distorted signal with different correction parameters used to multiply the distortion and modify the audio signal.

Fig. 6 plots the frequency in Hz on the x axis and the magnitude in dB on the y axis.

Three plots are shown in Fig. 6. In the first plot 601 the correction coefficient used was zero. In this case there was no modification of the audio signal.

In the second plot 603 the correction coefficient used was one. The wave representing the modification had the same magnitude as the intermodulation distortion but an inverse phase.

In the third plot 605 the correction coefficient used was 0.7. This value for the correction coefficient was obtained using examples of the disclosure. The wave representing the modification had 0.7 magnitude as the intermodulation distortion and an inverse phase.

The plots of Fig. 6 show that the examples of the disclosure provide reduces levels of distortion compared to providing no modification and for the trivial case of simply subtracting the distortion.

Experiments have been performed testing the examples described herein.

A first set of experiments used common loudspeaker models. A first model used for the loudspeaker model was an asymmetric loudspeaker in which the input-output relation was defined as:

$$r(n) = \gamma \left[ \frac{1}{1 + e^{-\theta r_1(n)}} - \frac{1}{2} \right]$$

Where, r1(n) = 1.5x(n) - 0.3x2(n) and y = 0.2, and θ is defined as:

$$\theta = \left\{ \begin{array}{ll} 4 & r_1(n) > 0 \\ \frac{1}{2} & r_1(n) \leq 0 \end{array} \right.$$

Using this model and the methods described herein an intermodulation distortion reduction of 34dB was obtained.

For a second set of experiments the loudspeaker model used was the symmetrical soft-clipping nonlinearity with $\tau = 0.3$. This can be written as:

$$r(n) = \left\{ \begin{array}{ll} \frac{2}{3\tau}x(n) & 0 \leq |x(n)| < \tau \\ \mathrm{sign}[x(n)] * \frac{3-[2-x(n)\tau]^2}{3} & \tau \leq |x(n)| < 2\tau \\ \mathrm{sign}[x(n)] & 2\tau \leq |x(n)| < 1 \end{array} \right.$$

Using this model and the methods described herein an intermodulation distortion reduction of 19.7 dB was obtained.

These experiments clearly demonstrate that examples of the disclosure can provide a reduction in intermodulation distortion in otoacoustic signals.

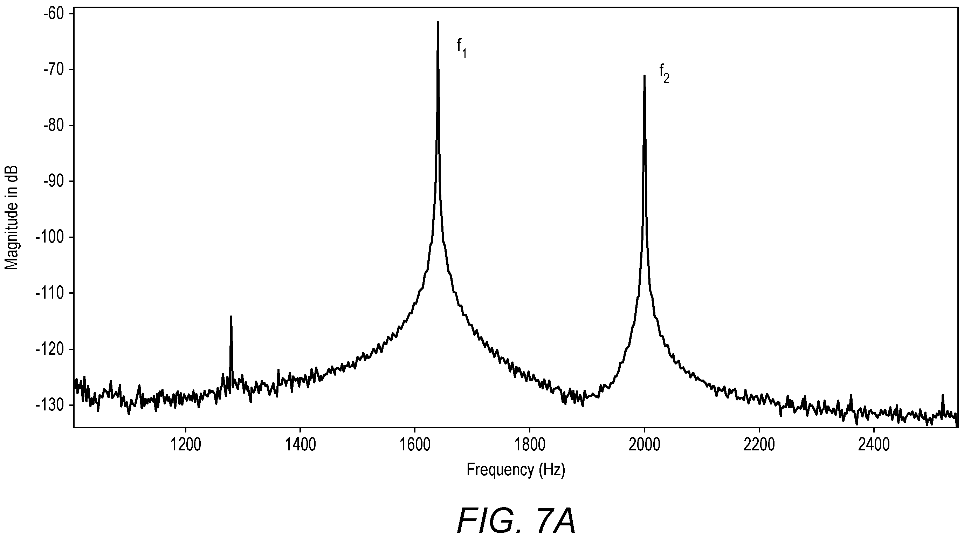

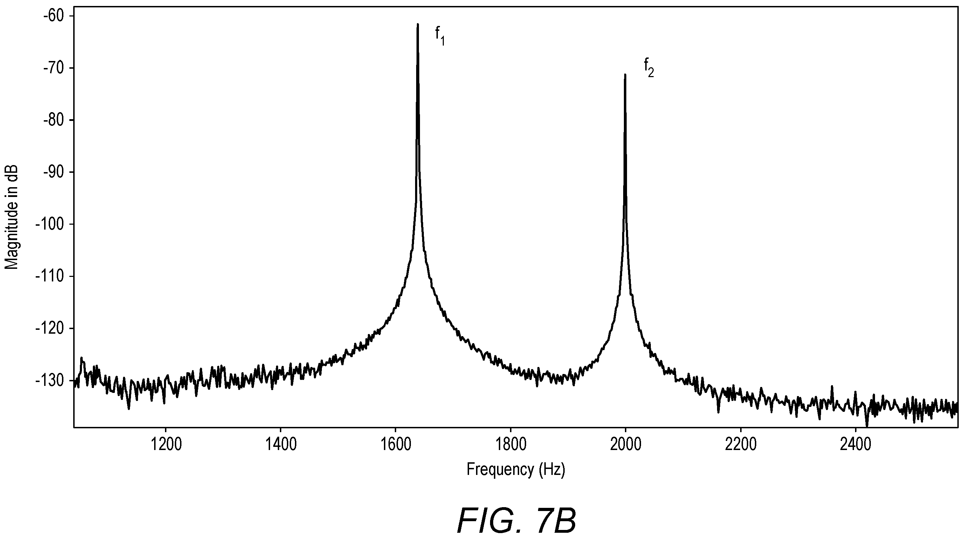

Figs. 7A and 7B show results obtained for examples of the disclosure. These results were obtained from experiments performed using a real microphone and loudspeaker located inside an earbud. A realistic ear canal response was simulated using a digital signal processing device. To obtain the results of Figs. 7A and 7B a miniDSP EARS device was used.

Figs. 7A and 7B show the power spectral estimation for an audio input signal where a single loudspeaker is used to playback the audio signal. Figs. 7A and 7B plot the frequency in Hz on the x axis and the magnitude in dB on the y axis.

The results shown in Fig. 7A were obtained without using examples of the disclosure and so there is no reduction in the intermodulation distortion. The results shown in Fig. 7B were obtained with using examples of the disclosure and so there is a reduction in the intermodulation distortion.

The comparison of these two figures shows a clear reduction in the intermodulation distortion at $2f_1$-$f_2$. In this example the intermodulation distortion has been completely cancelled. This therefore enables the single loudspeaker to be used to measure otoacoustic emissions.

Fig. 8 shows example devices 801 that could be used to measure otoacoustic emissions according to examples of the disclosure. The example devices 801 comprise earbuds. The earbuds comprise a housing 803 and an inner ear portion 805. The inner ear portion 805 is sized and shaped to fit into the inner ear of a user. When the earbuds are in use the inner ear portion is inserted into the inner ear of the user.

The housing 803 can be configured to house an apparatus or any other suitable control means for controlling the devices 801. An example apparatus is shown in Fig. 9.

Fig. 9 schematically illustrates an apparatus 901 that can be used to implement examples of the disclosure. In this example the apparatus 901 comprises a controller 903. The controller 903 can be a chip or a chip-set. In some examples the controller 903 can be provided within any suitable device such as earbuds or a device such as a smartphone that can be configured to communicate with the ear buds.

In the example of Fig. 9 the implementation of the controller 903 can be as controller circuitry. In some examples the controller 903 can be implemented in hardware alone, have certain aspects in software including firmware alone or can be a combination of hardware and software (including firmware).

As illustrated in Fig. 9 the controller 903 can be implemented using instructions that enable hardware functionality, for example, by using executable instructions of a computer program 909 in a general-purpose or special-purpose processor 905 that may be stored on a computer readable storage medium (disk, memory etc.) to be executed by such a processor 905.

The processor 905 is configured to read from and write to the memory 907. The processor 905 can also comprise an output interface via which data and/or commands are output by the processor 905 and an input interface via which data and/or commands are input to the processor 905.

The memory 907 stores a computer program 909 comprising computer program instructions (computer program code) that controls the operation of the controller 903 when loaded into the processor 905. The computer program instructions, of the computer program 909, provide the logic and routines that enables the controller 903. to perform the methods illustrated in the accompanying Figs. The processor 905 by reading the memory 907 is able to load and execute the computer program 909.

The apparatus 901 comprises:

at least one processor 905; and
at least one memory 907 including computer program code 911;
the at least one memory 907 and the computer program code 911 configured to, with
the at least one processor 905, cause the apparatus 901 at least to perform:

providing 201 an audio signal wherein the audio signal comprises two or more frequencies and is configured to be played back by a loudspeaker to enable otoacoustic emissions within a user's ear to be measured;
estimating 203 at least one interference component in an output of the loudspeaker playing back the obtained audio signal; and
using the estimated at least one interference component to estimate 205 a modification to be applied to at least one frequency of the audio signal to reduce the at least one interference component in the output of the loudspeaker.

As illustrated in Fig. 9, the computer program 909 can arrive at the controller 903 via any suitable delivery mechanism 913. The delivery mechanism 913 can be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid-state memory, an article of manufacture that comprises or tangibly embodies the computer program 909. The delivery mechanism can be a signal configured to reliably transfer the computer program 909. The controller 903 can propagate or transmit the computer program 909 as a computer data signal. In some examples the computer program 909 can be transmitted to the controller 903 using a wireless protocol such as Bluetooth, Bluetooth Low Energy, Bluetooth Smart, 6LoWPan (IP$_v$6 over low power personal area networks) ZigBee, ANT+, near field communication (NFC), Radio frequency identification, wireless local area network (wireless LAN) or any other suitable protocol.

The computer program 909 comprises computer program instructions for causing an apparatus 901 to perform at least the following or for performing at least the following:

providing 201 an audio signal wherein the audio signal comprises two or more frequencies and is configured to be played back by a loudspeaker to enable otoacoustic emissions within a user's ear to be measured;
estimating 203 at least one interference component in an output of the loudspeaker playing back the obtained audio signal; and
using the estimated at least one interference component to estimate 205 a modification to be applied to at least one frequency of the audio signal to reduce the at least one interference component in the output of the loudspeaker.

The computer program instructions can be comprised in a computer program 909, a non-transitory computer readable medium, a computer program product, a machine readable medium. In some but not necessarily all examples, the computer program instructions can be distributed over more than one computer program 909.

Although the memory 907 is illustrated as a single component/circuitry it can be implemented as one or more separate components/circuitry some or all of which can be integrated/removable and/or can provide permanent/semi-permanent/ dynamic/cached storage.

Although the processor 905 is illustrated as a single component/circuitry it can be implemented as one or more separate components/circuitry some or all of which can be integrated/removable. The processor 905 can be a single core or multi-core processor.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term 'circuitry' may refer to one or more or all of the following:

(a) hardware-only circuitry implementations (such as implementations in only analog and/or digital circuitry) and
(b) combinations of hardware circuits and software, such as (as applicable):

(i) a combination of analog and/or digital hardware circuit(s) with software/firmware and

(ii) any portions of hardware processor(s) with software (including digital signal processor(s)), software, and memory or memories that work together to cause an apparatus, such as a mobile phone or server, to perform various functions and

(c) hardware circuit(s) and or processor(s), such as a microprocessor(s) or a portion of a microprocessor(s), that requires software (for example, firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The blocks illustrated in Fig. 2 can represent steps in a method and/or sections of code in the computer program 909. The illustration of a particular order to the blocks does not necessarily imply that there is a required or preferred order for the blocks and the order and arrangement of the blocks can be varied. Furthermore, it can be possible for some blocks to be omitted.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one..." or by using "consisting".

In this description, the wording 'connect', 'couple' and 'communication' and their derivatives mean operationally connected/coupled/in communication. It should be appreciated that any number or combination of intervening components can exist (including no intervening components), i.e., so as to provide direct or indirect connection/coupling/communication. Any such intervening components can include hardware and/or software components.

As used herein, the term "determine/determining" (and grammatical variants thereof) can include, not least: calculating, computing, processing, deriving, measuring, investigating, identifying, looking up (for example, looking up in a table, a database or another data structure), ascertaining and the like. Also, "determining" can include receiving (for example, receiving information), accessing (for example, accessing data in a memory), obtaining and the like. Also, " determine/determining" can include resolving, selecting, choosing, establishing, and the like.

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain examples, those features may also be present in other examples whether described or not.

The term 'a', 'an' or 'the' is used in this document with an inclusive not an exclusive meaning. That is any reference to X comprising a/an/the Y indicates that X may comprise only one Y or may comprise more than one Y unless the context clearly indicates the contrary. If it is intended to use 'a', 'an' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

The above description describes some examples of the present disclosure however those of ordinary skill in the art will be aware of possible alternative structures and method features which offer equivalent functionality to the specific examples of such structures and features described herein above and which for the sake of brevity and clarity have been omitted from the above description. Nonetheless, the above description should be read as implicitly including reference to such alternative structures and method features which provide equivalent functionality unless such alternative structures or method features are explicitly excluded in the above description of the examples of the present disclosure.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus comprising means for:

   providing an audio signal wherein the audio signal comprises two or more frequencies and is configured to be played back by a loudspeaker to enable otoacoustic emissions within a user's ear to be measured;
   estimating at least one interference component in an output of the loudspeaker playing back the obtained audio signal; and
   using the estimated at least one interference component to estimate a modification to be applied to at least one frequency of the audio signal to reduce the at least one interference component in the output of the loudspeaker.

2. An apparatus as claim in claim 1, wherein the at least one interference component is estimated by at least one of:

   an output of a model of the loudspeaker; and
   processing a subset of a frequency range of the output of the model of the loudspeaker.

3. An apparatus as claimed in claim 2, wherein a machine learning program is used to model the loudspeaker.

4. An apparatus as claimed in claim 3, wherein the machine learning program comprises a neural network.

5. An apparatus as claimed in any preceding claim, wherein the at least one interference component comprises at least one of:

   a magnitude response;
   a phase response; and
   an intermodulation distortion.

6. An apparatus as claimed in any preceding claim, wherein estimating the modification to be applied to at least one frequency of the audio signal comprises determining a coefficient for multiplying at least one frequency of the audio signal.

7. An apparatus as claimed in any preceding claim, wherein a machine learning program is used to estimate the modification to be applied to at least one frequency of the audio signal.

8. An apparatus as claimed in claim 7, wherein the machine learning program comprises a neural network.

9. A method comprising:

   providing an audio signal wherein the audio signal comprises two or more frequencies and is configured to be played back by a loudspeaker to enable otoacoustic emissions within a user's ear to be measured;
   estimating at least one interference component in an output of the loudspeaker playing back the obtained audio signal; and
using the estimated at least one interference component to estimate a modification to be applied to at least one frequency of the audio signal to reduce the at least one interference component in the output of the loudspeaker.

10. A method as claimed in claim 9, wherein the at least one interference component is estimated by at least one of:

an output of a model of the loudspeaker; and
processing a subset of a frequency range of the output of the model of the loudspeaker.

11. A method as claimed in claim 10, wherein a machine learning program is used to model the loudspeaker.

12. A method as claimed in claim 11, wherein the machine learning program comprises a neural network.

13. A method as claimed in any of claims 9 to 12, wherein estimating the modification to be applied to at least one frequency of the audio signal comprises determining a coefficient for multiplying at least one frequency of the audio signal.

14. A method as claimed in any of claims 9 to 13, wherein a machine learning program is used to estimate the modification to be applied to at least one frequency of the audio signal.

15. A method as claimed in claim 14, wherein the machine learning program comprises a neural network.

*FIG. 1A*

EP 4 354 899 A1

FIG. 1B

EP 4 354 899 A1

Provide audio signal ~201

Estimate interference component ~203

Estimate modification ~205

*FIG. 2*

---

*301*

*313* *315* *317*

Polyphase filter

DFT

$X[k]$

*303*

$x[n]$

Loudspeaker model

*305*

*307*

$\tilde{y}[n]$

Polyphase filter

DFT

*309* *311*

$\tilde{Y}[2f_1\text{-}f_2]$

$\begin{bmatrix} f_1 \\ f_2 \end{bmatrix}$

$\begin{bmatrix} Re\{\tilde{Y}[2f_1\text{-}f_2]\} \\ Im\{\tilde{Y}[2f_1\text{-}f_2]\} \end{bmatrix}$

*319*

Correction model

$\alpha\tilde{Y}[2f_1\text{-}f_2]$

+

$\Sigma$

-

DFT

*321*

*FIG. 3*

*303*

*401*

*405*

*407*

$x[n]$   DAC   $\tilde{x}[t]$   $\Delta(.)$   $\tilde{y}[t]$

*403*

*409*

Loudspeaker model   $-$ $\Sigma$ $+$   ADC

$\tilde{y}[n]$

*305*   *411*

Error

**FIG. 4**

*319*

*503*

$$\begin{bmatrix} f_1 \\ f_2 \\ R(\tilde{Y}[2f_1\text{-}f_2]) \\ S(\tilde{Y}[2f_1\text{-}f_2]) \end{bmatrix}$$   Correction model   $-$ $\Sigma$ $+$   $\alpha$

*501*   *505*

Error

**FIG. 5**

*FIG. 6*

EP 4 354 899 A1

*FIG. 7A*

EP 4 354 899 A1

*FIG. 7B*

EP 4 354 899 A1

FIG. 8

FIG. 9

Europäisches
Patentamt

European
Patent Office

Office européen
des brevets

# EUROPEAN SEARCH REPORT

Application Number

EP 23 19 7871

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2022 0052601 A (SAMSUNG ELECTRONICS CO LTD [KR]) 28 April 2022 (2022-04-28) * paragraph [0057] - paragraph [0115]; figures 1-11 * ----- | 1-15 | INV. H04R3/04 H04R29/00 A61B5/12 |
| A | HSIAO WEI-CHEN ET AL: "Measuring Distortion-Product Otoacoustic Emission With a Single Loudspeaker in the Ear: Stimulus Design and Signal Processing Techniques", FRONTIERS IN DIGITAL HEALTH, vol. 3, 1 September 2021 (2021-09-01), pages 1-13, XP093088315, DOI: 10.3389/fdgth.2021.724539 * page 4 - page 7; figures 5,6 * ----- | 1-15 | ADD. H04R25/00 |

TECHNICAL FIELDS
SEARCHED      (IPC)

H04R

The present search report has been drawn up for all claims

| Place of search | Date of completion of the search | Examiner |
|---|---|---|
| Munich | 20 February 2024 | Righetti, Marco |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...................................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 19 7871

This annex lists the patent family members relating to the patent documents cited in the above-mentioned European search report.
The members are as contained in the European Patent Office EDP file on
The European Patent Office is in no way liable for these particulars which are merely given for the purpose of information.

20-02-2024

| Patent document cited in search report | Publication date | Patent family member(s) | | Publication date |
|---|---|---|---|---|
| KR 20220052601 A | 28-04-2022 | CN | 116348030 A | 27-06-2023 |
| | | EP | 4201324 A1 | 28-06-2023 |
| | | KR | 20220052601 A | 28-04-2022 |
| | | US | 2023225637 A1 | 20-07-2023 |
| | | WO | 2022085953 A1 | 28-04-2022 |

EPO FORM P0459

For more details about this annex : see Official Journal of the European Patent Office, No. 12/82